# EUROPEAN PATENT SPECIFICATION

(11) **EP 1 206 476 B1**
(45) Date of publication and mention of the grant of the patent: **04.01.2006**
(21) Application number: 00955256.3
(22) Date of filing: 26.07.2000
(51) Int. Cl.: C07H 17/08, A61K 31/70, A61P 31/04

(54) **9A-AZALIDES WITH ANTIBACTERIAL ACTIVITY**
9A-AZALIDE MIT ANTIBAKTERIELLER WIRKUNG
9A-AZALIDES A ACTIVITE ANTIBACTERIENNE

(30) Priority: 24.08.1999 US 382166
(43) Date of publication of application: 22.05.2002
(73) Proprietor: ABBOTT LABORATORIES, Abbott Park, IL 60064-6050 (US)
(72) Inventor: OR, Yat Sun, Cambridge, MA 02140 (US); KEYES, Robert F., Pleasant Prairie, WI 53258 (US); MA, Zhenkun, Gurnee, IL 60031 (US)
(74) Representative: Modiano, Micaela Nadia
(86) International application number: PCT/US2000/020363
(87) International publication number: WO 2001/014397

(56) References cited:
- EP-A- 0 109 253
- EP-A- 0 259 789
- WO-A-98/56801
- WO-A-98/56802
- WO-A-99/00124
- WO-A-99/00125
- US-A- 4 474 768
- US-A- 5 686 587
- WILKENING R R ET AL: "Novel Transannular Rearrangements of Azalide Iminoethers" TETRAHEDRON,NL,ELSEVIER SCIENCE PUBLISHERS, AMSTERDAM, vol. 53, no. 50, 15 December 1997 (1997-12-15), pages 16923-16944, XP004106498 ISSN: 0040-4020

## Description

### Technical Field

The instant invention relates to 9a-azalides which are antibacterial agents, compositions containing the compounds, processes for making the compounds, synthetic intermediates employed in the processes, and methods for treatment and prevention of bacterial infections.

### Background of The Invention

Macrolide antibacterial agents are widely used to treat and prevent bacterial infections. However, the discovery of bacterial strains which have resistance or insufficient susceptibility to macrolide antibacterial agents has promoted development of compounds with modified or improved profiles of antibiotic activity. One such class of compounds are azalides such as azithromycin, referred to in United States patents 4,474,768 and 4,517,359. Azalides are macrolide antibacterial agents with a core ring structurally similar to the erythronolide A or B ring except for the presence of a substituted or unsubstituted nitrogen moiety at the 9a position. Because of the potential for azalides to display modified or improved profiles of antibiotic activity, they are the subject of current research for their clinical potential**.**

PCT Application WO 98/56801, published December 17, 1998 discloses a series of 9a-(N-(alkyl))-azalide erythromycin A derivatives and a series of 9a-(N-(alkyl))-azalide 6-O-methylerythromycin A derivatives.

PCT Application WO 98/56802, published December 17, 1998 discloses a series of 9a-(N(H))-azalide erythromycin A derivatives and a series of 9a-(N(H))-azalide 6-O-methylerythromycin A derivatives.

PCT Application WO 99/00124, published January 7, 1999, discloses a series of 9a-(N(Rₙ))- azalide 3-thioxoerythromycin A derivatives and a series of 9a-(N(Rₙ))-azalide 6-O-methyl-3-oxoerythromycin A derivatives, wherein Rₙ is an optionally substituted alkyl or heteroalkyl.

PCT Application WO 99/00125, published January 7, 1999, discloses a series of 9a-(N(Rₙ))- azalide 3-oxoerythromycin A derivatives and a series of 9a-(N(Rₙ))-azalide 6-O-methyl-3-oxoerythromycin A derivatives, wherein Rₙ is an optionally substituted alkyl or heteroalkyl.

United States patent 5,686,587 discloses a synthesis of azithromycin comprising introducing a 9a-(N(H))- moiety to erythromycin A by oxime formation, Beckman rearrangement, reduction, and methylation.

EP-A-0 109 253 discloses antibacterial 4"-epi-9-deoxo-9a-methyl-9a-aza-homoerythromycin A which is the 4"-epimer of a previously reported erythromycin A derivative. In addition the intermediate 4"-epi-9-deoxo-9a-aza-9a-homoerythromycin A disclosed therein is the 4"-epimer of previously known 9-deoxo-9a-aza-9a-homoerythromycin A.

EP-A-0 259 789 discloses antibiotic metal complexes of N-methyl-11-aza-10-deoxo-10-dihydroerythromycin A or 11-aza-10-deoxo-10-dihydroerythromycin A.

Wilkening R.R., et al., Tetrahedron, vol. 53, no. 50, pages 16923-16944, disclose antibacterial 9-deoxo-9a-aza-9a-methyl-homoerythromycin A and the intermediate 9-deoxo-9a-aza-9a-homoerythromycin A.

### Summary of The Invention

In one embodiment of the present invention are disclosed compounds of formula (I) 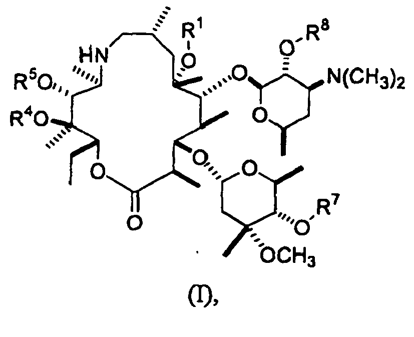 or pharmaceutically acceptable salts or prodrugs thereof, wherein
R¹ is selected from the group consisting of
(1) -C₃-C₁₂-alkenyl,
   and
(2) -C₃-C₁₂-alkynyl,
   wherein (1)-(2) can be optionally substituted with one, two, or three substituents independently selected from the group consisting of
   (a) halogen,
   (b) -OR⁹, wherein R⁹ is selected from the group consisting of
      (i) hydrogen,
      (ii) -C₁-C₁₂-alkyl,
      (iii) -C₂-C₁₂-heteroalkyl,
         wherein (ii) and (iii) can be optionally substituted with one, two, or three substituents independently selected from the group consisting of
         (1') aryl,
         (2') substituted aryl,
         (3') heteroaryl,
            and
         (4') substituted heteroaryl,
      (iv) aryl,
      (v) substituted aryl,
      (vi) heteroaryl,
         and
      (vii) substituted heteroaryl,
   (c) -NR¹¹R¹², wherein R¹¹ and R¹² are independently selected from the group consisting of
      (i) hydrogen,
      (ii) -C₁-C₁₂-alkyl,
      (iii) -C₂-C₁₂-heteroalkyl,
         wherein (ii) and (iii) can be optionally substituted with one, two, or three substituents independently selected from the group consisting of
         (1') aryl,
         (2') substituted aryl,
         (3') heteroaryl,
            and
         (4') substituted heteroaryl,
      (iv) aryl,
      (v) substituted aryl,
      (vi) heteroaryl,
         and
      (vii) substituted heteroaryl,

      or
      R¹¹ and R¹², together with the atom to which they are attached, form a heterocycloalkyl ring, wherein the heterocycloalkyl ring can be optionally substituted,
   (d) =N-O-R⁹, wherein R⁹ is defined above,
   (e) aryl,
   (f) substituted aryl,
   (g) heteroaryl,
   (h) substituted heteroaryl,
   (i) -C₃-C₈-cycloallcyl,
   (j) substituted -C₃-C₈-cycloalkyl,
   (k) heterocycloalkyl,
   (1) substituted heterocycloalkyl,
   (m) -NHC(O)R⁹, wherein R⁹ is defined above,
   (n) -NHC(O)OR¹⁰, wherein R¹⁰ is selected from the group consisting of
      (i) -C₁-C₁₂-alkyl,
      (ii) -C₁-C₁₂-heteroalkyl,
         wherein (i) and (ii) can be optionally substituted with one, two, or three substituents independently selected from the group consisting of
         (1') aryl,
         (2') substituted aryl,
         (3') heteroaryl,
            and
         (4') substituted heteroaryl,
      (iii) aryl,
      (iv) substituted aryl,
      (v) heteroaryl,
         and
      (vi) substituted heteroaryl,
   (o) -NHC(O)NR¹¹R¹², wherein R¹¹ and R¹² are defined above,
   (p) -OC(O)R¹⁰, wherein R¹⁰ is defined above,
   (q) -OC(O)OR¹⁰, wherein R¹⁰ is defined above,
   (r) -OC(O)NR¹¹R¹², wherein R¹¹ and R¹² are defined above,
   (s) -C(O)R⁹, wherein R⁹ is defined above,
   (t) -CO₂R⁹, wherein R⁹ is defined above,
      and
   (u) -C(O)NR¹¹R¹², wherein R¹¹ and R¹² are defined above;

R⁴ and R⁵ are hydrogen;
or
R⁴ and R⁵ together are -C(O)- or -(CH₂)ₓ-, wherein x is one, two, or three;
R⁷ is selected from the group consisting of
(1) hydrogen,
(2) -C₁-C₁₂-alkyl,
(3) -C₃-C₁₂-alkenyl,
(4) -C₃-C₁₂-alkynyl,
(5) -C₂-C₁₂-heteroalkyl,
(6) -C₄-C₁₂-heteroallcenyl,
(7) -C₄-C₁₂-heteroalkynyl,
   wherein (2)-(7) can be optionally substituted with one, two, or three substituents independently selected from the group consisting of
   (a) halo,
   (b) hydroxy,
   (c) -NR¹¹R¹², wherein R¹¹ and R¹² are defined above,
   (d) aryl,
   (e) substituted aryl,
   (f) heteroaryl,
      and
   (g) substituted heteroaryl,
(8) -C(O)R⁹, wherein R⁹ is defined above,
(9) -CO₂R⁹, wherein R⁹ is defined above,
   and
(11) -C(O)NR¹¹R¹², wherein R¹¹ and R¹² are defined above;

and
R⁸ is hydrogen or a hydroxy protecting group.

In another embodiment of the present invention are disclosed pharmaceutical compositions comprising a pharmaceutically effective amount of the compounds in combination with a pharmaceutically acceptable carrier.

The compounds of the present invention are useful for treating a bacterial infection in a mammal in need of such treatment by administering to the mammal a therapeutically effective amount of the compounds.

In still yet another embodiment of the present invention is disclosed a method for preparing the compounds,
the method comprising
(a) reacting a compound of formula (Ia) 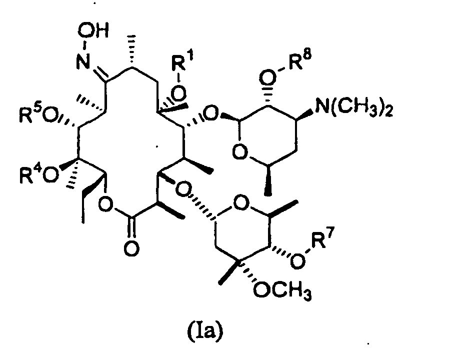
   wherein R¹, R⁴, R⁵, R⁷, and R⁸ are defined above,
   with an oxime activating agent;
(b) optionally reacting the product from step (a) with a reducing agent;
   and
(c) optionally deprotecting the product from step (b).

### Detailed Description of The Invention

The term "alkenyl," as used herein, refers to a monovalent straight or branched chain group containing at least one carbon-carbon double bond. The alkenyl groups can be optionally substituted.

The term "alkyl," as used herein, refers to saturated, straight or branched chain hydrocarbon radicals. Examples of alkyl radicals include methyl, ethyl, propyl, iso-propyl, n-butyl, *tert*-butyl, neo-pentyl, and n-hexyl. The alkyl groups can be optionally substituted.

The term "-C₁-C₃-alkylamino," as used herein, refers to a amino group, as defined herein wherein one hydrogen atom is replaced by a -C₁-C₃-alkyl group.
Examples of -C₁-C₃-alkylamino include methylamine, ethylamine, propylamine, and isopropylamine.

The term "-C₁-C₃-alkylthio," as used herein, refers to a -C₁-C₃-alkyl group, as defined herein, attached to the parent molecular group through a sulfur atom. Examples of -C₁-C₃-alkylthio include methyl sulfide, ethyl sulfide, propyl sulfide, and iso-propyl sulfide.

The term "alkoxy," as used herein, refers to an alkyl group, as previously defined, attached to the parent molecular group through an oxygen atom. Examples of alkoxy include methoxy, ethoxy, propoxy, isopropoxy, n-butoxy, tert-butoxy, neo-pentoxy and n-hexoxy. The alkoxy groups can be optionally substituted.

The term "alkynyl," as used herein, refers to a monovalent straight or branched chain group of two to six carbon atoms containing at least one carbon-carbon triple bond. Examples of alkynyl include ethynyl, propynyl, and butynyl. The alkynyl groups can be optionally substituted.

The term "amino," as used herein, refers to -NH₂.

The term "aprotic solvent," as used herein, refers to a solvent that is relatively inert to proton activity, i.e., not acting as a proton donor. Examples include hydrocarbons such as hexane and toluene, halogenated hydrocarbons such as dichloromethane, ethylene chloride, and chloroform, heterocyclic compounds such as tetrahydrofuran and N-methylprrolidinone, and ethers such as diethyl ether, bis-methoxymethyl ether. Such compounds are well known to those skilled in the art, and it will be obvious to those skilled in the art that individual solvents or mixtures thereof can be preferred for specific compounds and reaction conditions, depending upon such factors as the solubility of reagents, reactivity of reagents and preferred temperature ranges, for example. Further discussions of aprotic solvents can be found in organic chemistry textbooks or in specialized monographs, for example: Organic Solvents Physical Properties and Methods of Purification, 4th ed., edited by John A. Riddick, et al., Vol. II, in the Techniques of Chemistry Series, John Wiley & Sons, NY, 1986.

The term "aryl" as used herein refers to unsubstituted carbocyclic aromatic groups including phenyl, naphthyl, and anthracenyl.

The term " arylamino," as used herein, refers to a amino group, as defined herein wherein one hydrogen atom is replaced by an aryl group, as defined herein.

The term " aryloxy," as used herein, refers to an aryl group, as defined herein, attached to the parent molecular group through an oxygen atom.

The term " arylthio," as used herein, refers to an aryl-group, as defined herein, attached to the parent molecular group through a sulfur atom.

The term "azido," as used herein, refers to -N₃.

The term "benzyl," as used herein, refers to -CH₂C₆H₅.

The term " benzyloxy," as used herein, refers to a benzyl group, as defined herein, attached to the parent molecular group through an oxygen atom.

The term " benzylamino," as used herein, refers to a amino group, as defined herein wherein one hydrogen atom is replaced by a benzyl group, as defined herein.

The term " benzylthio," as used herein, refers to an benzyl group, as defined herein, attached to the parent molecular group through a sulfur atom.

The term "carboxaldehyde," as used herein, refers to -CHO.

The term "cyano," as used herein, refers to -CN.

The term "cycloalkyl," as used herein, refers to saturated carbocyclic groups having three to seven carbons such as cyclopropyl, cyclobutyl, cyclopentyl, cyclohexyl, and cycloheptyl.

The term "halo," as used herein, refers to -F, -Cl, -Br, and -I.

The term "heteroalkenyl," as used herein, refers to an alkenyl group having from four to twelve atoms, wherein at least atom is replaced with a group selected from -O-, =N-, -N(H)-, -N(CH₃)-, -C(O)-, -S(O)ₙ-, or a combination thereof, and the remaining atoms are carbon. The heteroalkenyl groups can be optionally substituted.

The term "heteroalkyl," as used herein, refers to an alkyl group having from two to twelve atoms, wherein at least atom is replaced with a group selected from -O-, =N-, - N(H)-, -N(CH₃)-, -C(O)-, -S(O)ₙ-, or a combination thereof, and the remaining atoms are carbon. The heteroalkyl groups can be optionally substituted.

The term "heteroalkynyl," as used herein, refers to an alkynyl group having from four to twelve atoms, wherein at least atom is replaced with a group selected from -O-, =N-, -N(H)-, -N(CH₃)-, -C(O)-, -S(O)ₙ-, or a combination thereof, and the remaining atoms are carbon. The heteroalkynyl groups can be optionally substituted.

The term "heteroaryl," as used herein, refers to a cyclic aromatic group having five or six ring atoms wherein at least one ring atom is selected from the group consisting of oxygen, sulfur, and nitrogen, and the remaining ring atoms are carbon. The nitrogen atoms can optionally be quaternized, and the sulfur atoms can optionally be oxidized. Heteroaryl groups of this invention include those derived from furan, imidazole, isothiazole, isoxazole, oxadiazole, oxazole, 1,2,3-oxadiazole, pyiazine, pyrazole, pyridazine, pyridine, pyrimidine, pyrroline, quinoline, thiazole, 1,3,4-thiadiazole, thiene, triazole, and tetrazole.

The term " heteroarylamino," as used herein, refers to a amino group, as defined herein wherein one hydrogen atom is replaced by a heteroaryl group, as defined herein.

The term " heteroaryloxy," as used herein, refers to a heteroaryl group, as defined herein, attached to the parent molecular group through an oxygen atom.

The term " heteroarylthio," as used herein, refers to a heteroaryl group, as defined herein, attached to the parent molecular group through a sulfur atom.

The term "heterocycloalkyl" as used herein, refers to a non-aromatic five-, six- or seven-membered ring or a bi- or tri-cyclic group comprising fused six-membered rings having between one and three heteroatoms independently selected from oxygen, sulfur and nitrogen wherein each 5-membered ring has zero to one double bonds and each six-membered ring has zero to 2 double bonds. The nitrogen and sulfur heteroatoms can optionally be oxidized, the nitrogen heteroatom can optionally be quaternized, and any of the above heterocyclic rings can be fused to a benzene ring. Representative heterocycles include, but are not limited to, pyrrolidinyl, pyrazolinyl, pyrazolidinyl, imidazolinyl, imidazolidinyl, piperidinyl, piperazinyl, oxazolidinyl, isoxazolidinyl, morpholinyl, thiazolidinyl, isothiazolidinyl, and tetrahydrofuryl. The heterocycloalkyl groups of this invention can be optionally substituted with one, two, three, or four substituents independently selected from -F, -Cl, -Br, -I, -OH, -NO₂, -CN, -C(O)-C₁-C₆-alkyl, -C(O)-aryl, -C(O)-heteroaryl, -CO₂-alkyl, -CO₂-aryl, -CO₂-heteroaryl, -CONH₂, -CONH-C₁-C₆-alkyl, -CONH-aryl, -CONH-heteroaryl, -OC(O)-C₁-C₆-alkyl, -OC(O)-aryl, -OC(O)-heteroaryl, -OCO₂-alkyl, -OCO₂-aryl, -OCO₂-heteroaryl, -OCONH₂, -OCONH-C₁-C₆-alkyl, -OCONH-aryl, -OCONH-heteroaryl, -NHC(O)-C₁-C₆-alkyl, -NHC(O)-aryl,-NHC(O)-heteroaryl, -NHCO₂-alkyl, -NHCO₂-aryl, -NHCO₂-heteroaryl, -NHCONH₂,-NHCONH-C₁-C₆-alkyl, -NHCONH-aryl, -NHCONH-heteroaryl, -SO₂-C₁-C₆-alkyl,-SO₂-aryl, -SO₂-heteroaryl, -SO₂NH₂, -SO₂NH-C₁-C₆-alkyl, -SO₂NH-aryl, -SO₂NH-heteroaryl, -C₁-C₆-alkyl, -C₃-C₆-cycloalkyl, -CF₃,-CH₂CF₃, -CHCl₂, -CH₂OH, -CH₂CH₂OH,-CH₂NH₂, -CH₂SO₂CH₃, aryl, heteroaryl, benzyl, benzyloxy, aryloxy, heteroaryloxy, -C₁-C₆-alkoxy, methoxymethoxy, methoxyethoxy; amino, benzylamino, arylamino, heteroarylamino, -C₁-C₃-alkylamino, thio, arylthio, heteroarylthio, benzylthio, -C₁-C₆-alkylthio, or methylthiomethyl.

The term "hydroxy," as used herein, refers to -OH:

The term "hydroxy protecting group", as used herein, refers to an easily removable group to which are known in the art to protect a hydroxyl group against undesirable reaction during synthetic procedures and to be selectively removable. The use of hydroxy-protecting groups is well known in the art for protecting groups against undesirable reactions during a synthetic procedure and many such protecting groups are known, cf, for example, T.H. Greene and P.G.M. Wuts, Protective Groups in Organic Synthesis, 2nd edition, John Wiley & Sons, New York (1991). Examples of hydroxy-protecting groups include, methylthiomethyl, *tert*-dimethylsilyl, *tert*-butyldiphenylsilyl, acyl substituted with an aromatic group, and the like.

The term "methoxymethoxy," as used herein, refers to-OCH₂OCH₃.

The term "methoxyethoxy," as used herein, refers to-OCH₂OCH₂CH₃.

The term "methylthiomethyl," as used herein, refers to-CH₂SCH₃.

The term "oxo," as used herein, refers to a group formed by the replacement of two hydrogen atoms on the same carbon atom of an alkyl group, as defined above, with a single oxygen atom and is exemplified by a carbonyl group.

A the term "protected hydroxy" refers to a hydroxy group protected with a hydroxy protecting group, as defined above, such as benzoyl, acetyl, trimethylsilyl, triethylsilyl, or methoxymethyl groups.

The term "substituted aryl," as used herein, refers to an aryl group, as defined herein, substituted by independent replacement of one, two or three of the hydrogen atoms thereon with -F, -Cl, -Br, -I, -OH, -NO₂, -CN, -C(O)-C₁-C₆-alkyl, -C(O)-aryl, -C(O)-heteroaryl, -CO₂-alkyl, -CO₂-aryl, -CO₂-heteroaryl, -CONH₂, -CONH-C₁-C₆-alkyl, - CONH-aryl, -CONH-heteroaryl, -OC(O)-C₁-C₆-alkyl, -OC(O)-aryl, -OC(O)-heteroaryl, - OCO₂-alkyl, -OCO₂-aryl, -OCO₂-heteroaryl, -OCONH₂, -OCONH-C₁-C₆-alkyl, - OCONH-aryl, -OCONH-heteroaryl, -NHC(O)-C₁-C₆-alkyl, -NHC(O)-aryl, -NHC(O)-heteroaryl, -NHCO₂-alkyl, -NHCO₂-aryl, -NHCO₂-heteroaryl, -NHCONH₂, -NHCONH-C₁-C₆-alkyl, -NHCONH-aryl, -NHCONH-heteroaryl, -SO₂-C₁-C₆-alkyl, -SO₂-aryl, -SO₂-heteroaryl, -SO₂NH₂, -SO₂NH-C₁-C₆-alkyl, -SO₂NH-aryl, -SO₂NH-heteroaryl, -C₁-C₆-alkyl, -C₃-C₆-cycloalkyl, -CF₃, -CH₂CF₃, -CHCl₂, -CH₂OH, -CH₂CH₂OH, -CH₂NH₂, -CH₂SO₂CH₃, aryl, heteroaryl, benzyl, benzyloxy, aryloxy, heteroaryloxy, -C₁-C₆-alkoxy, methoxymethoxy, methoxyethoxy, amino, benzylamino, arylamino, heteroarylamino, -C₁-C₃-alkylamino, thio, arylthio, heteroarylthio, benzylthio, -C₁-C₆-alkylthio, or methylthiomethyl.

The term "substituted heteroaryl" as used herein refers to a heteroaryl group as defined herein substituted by independent replacement of one, two or three of the hydrogen atoms thereon with -F, -Cl, -Br, -I, -OH, -NO₂, -CN, -C(O)-C₁-C₆-alkyl, -C(O)-aryl, -C(O)-heteroaryl, -CO₂-alkyl, -CO₂-aryl, -CO₂-heteroaryl, -CONH₂, -CONH-C₁-C₆-alkyl, -CONH-aryl, -CONH-heteroaryl, -OC(O)-C₁-C₆-alkyl, -OC(O)-aryl, -OC(O)-heteroaryl, -OCO₂-alkyl, -OCO₂-aryl, -OCO₂-heteroaryl, -OCONH₂, -OCONH-C₁-C₆-alkyl, -OCONH-aryl, -OCONH-heteroaryl, -NHC(O)-C₁-C₆-alkyl, -NHC(O)-aryl, -NHC(O)-heteroaryl, -NHCO₂-alkyl, -NHCO₂-aryl, -NHCO₂-heteroaryl, -NHCONH₂, -NHCONH-C₁-C₆-alkyl, -NHCONH-aryl, -NHCONH-heteroaryl, -SO₂-C₁-C₆-alkyl, -SO₂-aryl, -SO₂-heteroaryl, -SO₂NH₂, -SO₂NH-C₁-C₆-alkyl, -SO₂NH-aryl, -SO₂NH-heteroaryl, -C₁-C₆-alkyl, -C₃-C₆-cycloalkyl, -CF₃, -CH₂CF₃, -CHCl₂, -CH₂OH, -CH₂CH₂OH, -CH₂NH₂, -CH₂S0₂CH₃, aryl, heteroaryl, benzyl, benzyloxy, aryloxy, heteroaryloxy, -C₁-C₆-alkoxy, methoxymethoxy, methoxyethoxy, amino, benzylamino, arylamino, heteroarylamino, -C₁-C₃-alkylamino, thio, arylthio, heteroarylthio, benzylthio, -C₁-C₆-alkylthio, or methylthiomethyl.

The term "substituted heterocycloalkyl," as used herein, refers to a heterocycloalkyl group, as defined above, substituted by independent replacement of one, two or three of the hydrogen atoms thereon with -F, -Cl, -Br, -I, -OH, -NO₂, -CN, -C(O)-C₁-C₆-alkyl, -C(O)-aryl, -C(O)-heteroaryl, -CO₂-alkyl, -CO₂-aryl, -CO₂-heteroaryl, -CONH₂, -CONH-C₁-C₆-alkyl, -CONH-aryl, -CONH-heteroaryl, -OC(O)-C₁-C₆-alkyl, -OC(O)-aryl, - OC(O)-heteroaryl, -OCO₂-alkyl, -OCO₂-aryl, -OCO₂-heteroaryl, -OCONH₂, -OCONH-C₁-C₆-alkyl, -OCONH-aryl, -OCONH-heteroaryl, -NHC(O)-C₁-C₆-alkyl, -NHC(O)-aryl, -NHC(O)-heteroaryl, -NHCO₂-alkyl, -NHCO₂-aryl, -NHCO₂-heteroaryl, -NHCONH₂,-NHCONH-C₁-C₆-alkyl, -NHCONH-aryl, -NHCONH-heteroaryl, -SO₂-C₁-C₆-alkyl,-SO₂-aryl, -SO₂-heteroaryl, -SO₂NH₂, -SO₂NH-C₁-C₆-alkyl, -SO₂NH-aryl, -SO₂NH-heteroaryl, -C₁-C₆-alkyl, -C₃-C₆-cycloalkyl, -CF₃, -CH₂CF₃, -CHCl₂, -CH₂OH,-CH₂CH₂OH, -CH₂NH₂, -CH₂SO₂CH₃, aryl, heteroaryl, benzyl, benzyloxy, aryloxy, heteroaryloxy, alkoxy, methoxymethoxy, methoxyethoxy, amino, benzylamino, arylamino, heteroarylamino, -C₁-C₃-alkylamino, thio, arylthio, heteroarylthio, benzylthio, alkylthio, or methylthiomethyl.

The term "thio," as used herein, refers to -SH.

Numerous asymmetric centers exist in the compounds of the present invention. Except where otherwise noted, the present invention contemplates the various stereoisomers and mixtures thereof. Accordingly, whenever a bond is represented by a wavy line or a straight line, it is intended that a mixture of stereo-orientations or an individual isomer of unassigned orientation can be present.

The term "pharmaceutically acceptable prodrugs," as used herein refers to, those prodrugs of the compounds of the present invention which are, within the scope of sound medical judgment, suitable for use in contact with the tissues of humans and lower animals with undue toxicity, irritation, allergic response, and the like, commensurate with a reasonable benefit/risk ratio, and effective for their intended use, as well as the zwitterionic forms, where possible, of the compounds of the invention.

The term "prodrug," as used herein, represents compounds which are rapidly transformed *in vivo* to parent compounds defined above, for example, by hydrolysis in blood. A thorough discussion is provided in T. Higuchi and V. Stella, Prodrugs as Novel Delivery Systems, Vol. 14 of the A.C.S. Symposium Series, and in Edward B. Roche, ed., Bioreversible Carriers in Drug Design, American Pharmaceutical Association and Pergamon Press, 1987.

The term "pharmaceutically acceptable salt," as used herein, refers to those salts which are, within the scope of sound medical judgment, suitable for use in contact with the tissues of humans and lower animals without undue toxicity, irritation, and allergic response and are commensurate with a reasonable benefit/risk ratio. Pharmaceutically acceptable salts are well known in the art. For example, S. M. Berge, et al. describe pharmaceutically acceptable salts in detail in J. Pharmaceutical Sciences, 66: 1-19 (1977), the relevant portions of which are incorporated herein by reference. The salts can be prepared in situ during the final isolation and purification of the compounds of the invention or separately by reacting a free base group with a suitable organic acid. Examples of pharmaceutically acceptable, nontoxic acid addition salts are salts of an amino group formed with inorganic acids such as hydrochloric acid, hydrobromic acid, phosphoric acid, sulfuric acid, and perchloric acid or with organic acids such as acetic acid, oxalic acid, maleic acid, tartaric acid, citric acid, succinic acid, or malonic acid or by using other methods used in the art such as ion exchange. Other pharmaceutically acceptable salts include adipate, alginate, ascorbate, aspartate, benzenesulfonate, benzoate, bisulfate, borate, butyrate, camphorate, camphorsulfonate, citrate, cyclopentanepropionate, digluconate, dodecylsulfate, ethanesulfonate; formate, fumarate, glucoheptonate, glycerophosphate, gluconate, hemisulfate, heptanoate, hexanoate, hydroiodide, 2-hydroxyethanesulfonate, lactobionate, lactate, laureate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, picrate, pivalate, propionate, stearate, succinate, sulfate, tartrate, thiocyanate, p-toluenesulfonate, undecanoate, and valerate. Representative alkali or alkaline earth metal salts include sodium, lithium, potassium, calcium, and magnesium. Further pharmaceutically acceptable salts include, when appropriate, nontoxic ammonium, quaternary ammonium, and amine cations formed using counterions such as halide, hydroxide, carboxylate, sulfate, phosphate, nitrate, loweralkyl sulfonate, and aryl sulfonate.

The pharmaceutical compositions of the present invention comprise a therapeutically effective amount of a compound of the present invention formulated together with one or more pharmaceutically acceptable carriers. As used herein, the term "pharmaceutically acceptable carrier" means a non-toxic, inert solid, semi-solid or liquid filler, diluent, encapsulating material or formulation auxiliary of any type. Some examples of materials which can serve as pharmaceutically acceptable carriers are sugars such as lactose, glucose and sucrose; starches such as corn starch and potato starch; cellulose and its derivatives such as sodium carboxymethyl cellulose, ethyl cellulose and cellulose acetate; powdered tragacanth; malt; gelatin; talc; excipients such as cocoa butter and suppository waxes; oils such as peanut oil, cottonseed oil; safflower oil; sesame oil; olive oil; corn oil and soybean oil; glycols; such a propylene glycol; esters such as ethyl oleate and ethyl laureate; agar; buffering agents such as magnesium hydroxide and aluminum hydroxide; alginic acid; pyrogen-free water; isotonic saline; Ringer's solution; ethyl alcohol, and phosphate buffer solutions, as well as other non-toxic compatible lubricants such as sodium lauryl sulfate and magnesium stearate, as well as coloring agents, releasing agents, coating agents, sweetening, flavoring and perfuming agents, preservatives and antioxidants can also be present in the composition, according to the judgment of the formulator. The pharmaceutical compositions of this invention can be administered to humans and other animals orally, rectally, parenterally, intracisternally, intravaginally, intraperitoneally, topically (as by powders, ointments, or drops), bucally, or as an oral or nasal spray.

Liquid dosage forms for oral administration include pharmaceutically acceptable emulsions, microemulsions, solutions, suspensions, syrups and elixirs. In addition to the active compounds, the liquid dosage forms can contain inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethylformamide, oils (in particular, cottonseed, groundnut, corn, germ, olive, castor, and sesame oils), glycerol, tetrahydrofurfuryl alcohol, polyethylene glycols and fatty acid esters of sorbitan, and mixtures thereof. Besides inert diluents, the oral compositions can also include adjuvants such as wetting agents, emulsifying and suspending agents, sweetening, flavoring, and perfuming agents.

Injectable preparations, for example, sterile injectable aqueous or oleaginous suspensions can be formulated according to the known art using suitable dispersing or wetting agents and suspending agents. The sterile injectable preparation can also be a sterile injectable solution, suspension or emulsion in a nontoxic parenterally acceptable diluent or solvent, for example, as a solution in 1,3-butanediol. Among the acceptable vehicles and solvents that can be employed are water, Ringer's solution, U.S.P. and isotonic sodium chloride solution. In addition, sterile, fixed oils are conventionally employed as a solvent or suspending medium. For this purpose any bland fixed oil can be employed including synthetic mono- or diglycerides. In addition, fatty acids such as oleic acid are used in the preparation of injectables.

The injectable formulations can be sterilized, for example, by filtration through a bacterial-retaining filter, or by incorporating sterilizing agents in the form of sterile solid compositions which can be dissolved or dispersed in sterile water or other sterile injectable medium prior to use.

In order to prolong the effect of a drug, it is often desirable to slow the absorption of the drug from subcutaneous or intramuscular injection. This can be accomplished by the use of a liquid suspension of crystalline or amorphous material with poor water solubility. The rate of absorption of the drug then depends upon its rate of dissolution which, in turn, can depend upon crystal size and crystalline form. Alternatively, delayed absorption of a parenterally administered drug form is accomplished by dissolving or suspending the drug in an oil vehicle. Injectable depot forms are made by forming microencapsule matrices of the drug in biodegradable polymers such as polylactide-polyglycolide. Depending upon the ratio of drug to polymer and the nature of the particular polymer employed, the rate of drug release can be controlled. Examples of other biodegradable polymers include poly(orthoesters) and poly(anhydrides). Depot injectable formulations are also prepared by entrapping the drug in liposomes or microemulsions which are compatible with body tissues.

Compositions for rectal or vaginal administration are preferably suppositories which can be prepared by mixing the compounds of this invention with suitable nonirritating excipients or carriers such as cocoa butter, polyethylene glycol or a suppository wax which are solid at ambient temperature but liquid at body temperature and therefore melt in the rectum or vaginal cavity and release the active compound.

Solid dosage forms for oral administration include capsules, tablets, pills, powders, and granules. In such solid dosage forms, the active compound is mixed with at least one inert, pharmaceutically acceptable excipient or carrier such as sodium citrate or dicalcium phosphate and/or a) fillers or extenders such as starches, lactose, sucrose, glucose, mannitol, and silicic acid, b) binders such as, for example, carboxymethylcellulose, alginates, gelatin, polyvinylpyrrolidinone, sucrose, and acacia, c) humectants such as glycerol, d) disintegrating agents such as agar-agar, calcium carbonate, potato or tapioca starch, alginic acid, certain silicates, and sodium carbonate, e) solution retarding agents such as paraffin, f) absorption accelerators such as quaternary ammonium compounds, g) wetting agents such as, for example, cetyl alcohol and glycerol monostearate, h) absorbents such as kaolin and bentonite clay, and i) lubricants such as talc, calcium stearate, magnesium stearate, solid polyethylene glycols, sodium lauryl sulfate, and mixtures thereof. In the case of capsules, tablets and pills, the dosage form can also comprise buffering agents.

Solid compositions of a similar type can also be employed as fillers in soft and hard-filled gelatin capsules using such excipients as lactose or milk sugar as well as high molecular weight polyethylene glycols and the like.

The solid dosage forms of tablets, dragees, capsules, pills, and granules can be prepared with coatings and shells such as enteric coatings and other coatings well known in the pharmaceutical formulating art. They can optionally contain opacifying agents and can also be of a composition that they release the active ingredient(s) only, or preferentially, in a certain part of the intestinal tract, optionally, in a delayed manner. Examples of embedding compositions which can be used include polymeric substances and waxes.

Solid compositions of a similar type can also be employed as fillers in soft and hard-filled gelatin capsules using such excipients as lactose or milk sugar as well as high molecular weight polethylene glycols and the like.

The active compounds can also be in micro-encapsulated form with one or more excipients as noted above. The solid dosage forms of tablets, dragees, capsules, pills, and granules can be prepared with coatings and shells such as enteric coatings, release controlling coatings and other coatings well known in the pharmaceutical formulating art. In such solid dosage forms the active compound can be admixed with at least one inert diluent such as sucrose, lactose or starch. Such dosage forms can also comprise, as is normal practice, additional substances other than inert diluents, e.g., tableting lubricants and other tableting aids such a magnesium stearate and microcrystalline cellulose. In the case of capsules, tablets and pills, the dosage forms can also comprise buffering agents. They can optionally contain opacifying agents and can also be of a composition that they release the active ingredient(s) only, or preferentially, in a certain part of the intestinal tract, optionally, in a delayed manner. Examples of embedding compositions which can be used include polymeric substances and waxes.

Dosage forms for topical or transdermal administration of a compound of this invention include ointments, pastes, creams, lotions, gels, powders, solutions, sprays, inhalants or patches. The active component is admixed under sterile conditions with a pharmaceutically acceptable carrier and any needed preservatives or buffers as can be required. Ophthalmic formulation, ear drops, eye ointments, powders and solutions are also contemplated as being within the scope of this invention.

The ointments, pastes, creams and gels can contain, in addition to an active compound of this invention, excipients such as animal and vegetable fats, oils, waxes, paraffins, starch, tragacanth, cellulose derivatives, polyethylene glycols, silicones, bentonites, silicic acid, talc and zinc oxide, or mixtures thereof.

Powders and sprays can contain, in addition to the compounds of this invention, excipients such as lactose, talc, silicic acid, aluminum hydroxide, calcium silicates and polyamide powder, or mixtures of these substances. Sprays can additionally contain customary propellants such as chlorofluorohydrocarbons.

Transdermal patches have the added advantage of providing controlled delivery of a compound to the body. Such dosage forms can be made by dissolving or dispensing the compound in the proper medium. Absorption enhancers can also be used to increase the flux of the compound across the skin. The rate can be controlled by either providing a rate controlling membrane or by dispersing the compound in a polymer matrix or gel.

According to the methods of treatment, bacterial infections are treated or prevented in a patient such as a human or lower mammal by administering to the patient a therapeutically effective amount of a compound of the invention, in such amounts and for such time as is necessary to achieve the desired result. By a "therapeutically effective amount" of a compound of the invention is meant a sufficient amount of the compound to treat bacterial infections, at a reasonable benefit/risk ratio applicable to any medical treatment. It will be understood, however, that the total daily usage of the compounds and compositions of the present invention will be decided by the attending physician within the scope of sound medical judgment. The specific therapeutically effective dose level for any particular patient will depend upon a variety of factors including the disorder being treated and the severity of the disorder; the activity of the specific compound employed; the specific composition employed; the age, body weight, general health, sex and diet of the patient; the time of administration, route of administration, and rate of excretion of the specific compound employed; the duration of the treatment; drugs used in combination or coincidental with the specific compound employed; and like factors well known in the medical arts.

The total daily dose of the compounds of this invention administered to a human or other mammal in single or in divided doses can be in amounts, for example, from 0.01 to 50 mg/kg body weight or more usually from 0.1 to 25 mg/kg body weight. Single dose compositions can contain such amounts or submultiples thereof to make up the daily dose. In general, treatment regimens according to the present invention comprise administration to a patient in need of such treatment from about 10 mg to about 1000 mg of the compound(s) of this invention per day in single or multiple doses.

Preferred compounds of the invention include:
Compound of formula (I): R⁴ and R⁵ are hydrogen.
Compound of formula (I): R⁴ and R⁵ together are -C(O)-.
Compound of formula (I): R¹ is -CH₂-CH=CH₂.
Compound of formula (I): R¹ is -CH₂-CH=CH-(3-quinolinyl).

Specific compounds of the invention include:
Compound of formula (I): R¹ is -CH₂CH=CH₂, R⁴ is hydrogen, R⁵ is hydrogen, R⁷ is hydrogen, R⁸ is hydrogen,

### Determination of Biological Activity

### In Vitro Assay of Antibacterial Activity

Representative compounds of the present invention were assayed *in vitro* for antibacterial activity as follows: Twelve petri dishes containing successive aqueous dilutions of the test compound mixed with 10 mL of sterilized Brain Heart Infusion (BHI) agar (Difco 0418-01-5) were prepared. Each plate was inoculated with 1:100 (or 1:10 for slow-growing strains, such as Micrococcus and Streptococcus) dilutions of up to 32 different microorganisms, using a Steers replicator block. The inoculated plates were incubated at 35-37 °C for 20 to 24 hours. In addition, a control plate, using BHI agar containing no test compound, was prepared and incubated at the beginning and end of each test.

An additional plate containing a compound having known susceptibility patterns for the organisms being tested and belonging to the same antibiotic class as the test compound was also prepared and incubated as a further control, as well as to provide test-to-test comparability. Erythromycin A was used for this purpose.

After incubation, each plate was visually inspected. The minimum inhibitory concentration (MIC) was defined as the lowest concentration of drug yielding no growth, a slight haze, or sparsely isolated colonies on the inoculum spot as compared to the growth control. The results of this assay, shown below in Table 1, demonstrate the antibacterial activity of the compounds of the invention.

| Microorganism | Code |
|---|---|
| Staphylococcus aureus ATCC 6538P | AA |
| Staphylococcus aureus A-5177 | BB |
| Staphylococcus aureus A-5278 | CC |
| Staphylococcus aureus CMX 642A | DD |
| Staphylococcus aureus NCTC 10649M | EE |
| Staphylococcus aureus CMX 553 | FF |
| Staphylococcus aureus 1775 | GG |
| Staphylococcus epidermidis 3519 | HH |
| Enterococcusfaecium ATCC X043 | II |
| Streptococcus bovis A-5169 | JJ |
| Streptococcus agalactiae CMX 508 | KK |
| Streptococcus pyogenes EES61 | LL |
| Streptococcus pyogenes 930 | MM |
| Streptococcus pyogenes PIU 2548 | NN |
| Micrococcusluteus ATCC 9341 | 00 |
| Micrococcusluteus ATCC 4698 | PP |
| Escherichiacoli JUHL | QQ |
| Escherichiacoli SS | RR |
| Escherichiacoli DC-2 | SS |
| Candida albicans CCH 442 | TT |
| Mycobacterium smegmatis ATCC 114 | UU |
| Nocardia Asteroides ATCC 99700 | VV |
| Haemophilislnfluenzae DILL AMP R | WW |
| Streptococcus Pneumonia ATCC 6303 | XX |
| Streptococcus Pneumonia GYR 1171 | YY |
| Streptococcus Pneumonia 5979 | ZZ |
| Streptococcus Pneumonia 5649 | ZA |

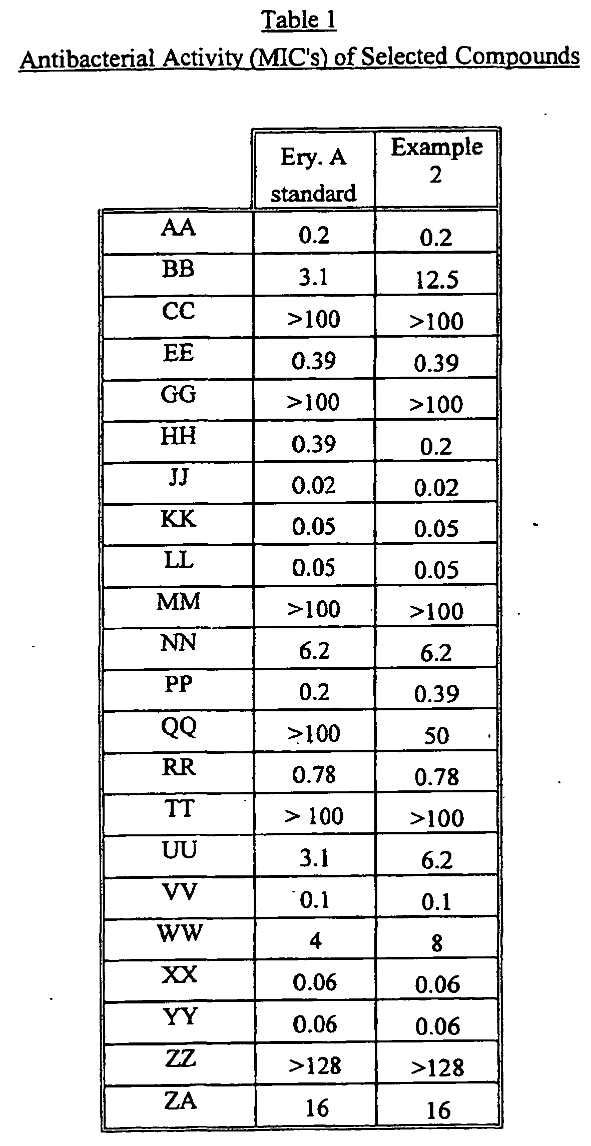

### Synthetic Methods

### Abbreviations

Abbreviations which have been used in the descriptions of the schemes and the examples that follow are: Ac for acetate; Bz for benzoyl; dba for dibenzylidine acetone; CDI for carbonyldiimidazole; DCM for dichloromethane; DMA for N,N-dimethylacetamide; DMAP for 4-(N,N-dimethylamino)pyridine; DME for dimethoxyethane; DMF for N,N-dimethylformamide; DMS for dimethylsulfide; DMSO for dimethylsulfoxide; dppb for 1,4-bis(diphenylphosphino)butane; EDCI for 1-[3-(dimethylamino)propyl]-3-ethylcarbodiimide hydrochloride; HMPA for hexamethylphosphoramide; MTBE for methyl *tert*-butyl ether; TEA for triethylamine; TFA for trifluoroacetic acid; THF for tetrahydrofuran; and TBAB for tetrabutylammonium bromide.

The compounds and processes of the present invention will be better understood in connection with the following synthetic schemes which illustrate the methods by which the compounds of the invention can be prepared. The compounds defined above can be prepared by a variety of synthetic routes. Representative procedures are shown below in Schemes 1-7. The groups R¹, R⁴, R⁵, R⁶, R⁷, and R⁸ are defined above, and the groups X¹, X² and X³ are defined below. It will be readily apparent to one of ordinary skill in the art that the compounds defined above can be synthesized by substitution of the appropriate reactants and agents in the syntheses shown below. It will also be apparent to one skilled in the art that the selective protection and deprotection steps, as well as the order of the steps themselves, can be carried out in varying order, depending on the nature of R¹, R⁴, R⁵, R⁶, R⁷, and R⁸, to successfully complete the syntheses of compounds defined above. A thorough discussion of protecting groups is provided in Greene and Wuts, Protective Groups in Organic Synthesis, 2nd ed., John Wiley & Son, Inc., 1991.

The groups R¹, R⁷, R⁴ and R⁵ together, when each is other than hydrogen, can be introduced to the erythronolide ring (for R¹, and R⁴ and R⁵ together) or cladinose ring (for R⁷) from precursor compounds R¹-X¹, X¹-(CH₂)ₓ-X¹ or X¹-C(O)-X¹, and R⁷-X¹, respectively. In each case, X¹ is an attachment group comprising a halo or sulfonate leaving group or a carbonyl activating group. The precursor compounds are commercially available or can be prepared from commercially available starting materials. For example, compounds containing an alcohol group can be elaborated to alkyl, alkenyl, alkynyl, aldehyde, ether, acid halide, ester, amide, amine, oxime, thioalkoxide, sulfinyl, sulfonyl, or carboxylic acid-containing groups by means well known in the art. Many of these groups can be further elaborated to other groups such as carbonates, carbamates, or ureas. Functional group transformations useful for preparing precursor compounds R¹-X¹, X¹-(CH₂)ₓ-X¹ or X¹-C(O)-X¹, and R⁷-X¹ are disclosed in Larock, "Comprehensive Organic Transformations. A Guide to Functional Group Preparations," VCH Publishers, New York (1989). The introduction of these groups to the erythronolide or cladinose ring is discussed in the schemes.

Compounds of formula (1) in Scheme 1 can be prepared from erythromycin A. The synthesis is described in United States Patents 4,990,602, 4,331,803, and 4,670,549. The C-9-carbonyl of erythromycin A can be protected as an oxime. Preferred protecting groups of the C-9-carbonyl are =N-O-R^{x} or =N-O-C(R^{y})(R^{z})(OR^{x}), wherein R^{x} is (a) -C₁-C₁₂-alkyl, (b) -C₁-C₁₂-alkyl substituted with aryl, (c) -C₁-C₁₂-alkyl substituted with substituted aryl, (d) -C₁-C₁₂-alkyl substituted with heteroaryl, (e) -C₁-C₁₂-alkyl substituted with substituted heteroaryl, (f) -C₃-C₁₂-cycloalkyl, or (g) -Si(R^{a})₃, wherein R^{a} is -C₁-C₁₂-alkyl or aryl, and wherein R^{y} and R^{z} are independently (a) hydrogen, (b) -C₁-C₁₂-alkyl, (c) -C₁-C₁₂-alkyl substituted with aryl, or (d) -C₁-C₁₂-alkyl substituted with substituted aryl, or R^{y} and R^{z} taken together with the carbon to which they are attached form a -C₃-C₁₂-cycloalkyl ring. A particularly preferred carbonyl protecting group for the C-9-carbonyl of erythromycin A is O-(1-isopropoxycyclohexyl) oxime, wherein R^{y} and R^{z} together are cyclohexyl and R^{x} is isopropyl.

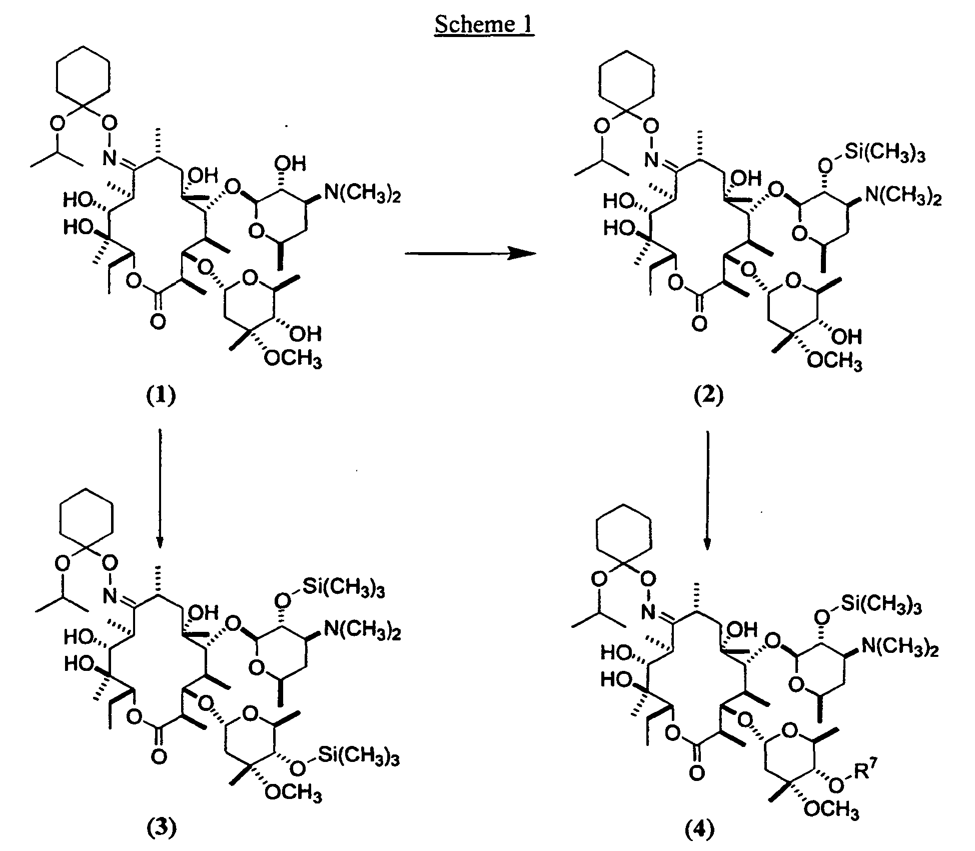

As shown in Scheme 1, the 2'-hydroxy of the desosamine ring and 4"-hydroxy of the cladinose ring of compounds of formula (**1**) can be protected sequentially or simultaneously by treatment of the same with hydroxy protecting group precursors to provide compounds of formulas (**2**) or (**3**), respectively. Hydroxy protecting group precursors include, acetic anhydride, benzoic anhydride, hexamethyldisilazane, a trialkylsilyl or triarylsilyl halide, or benzyl chloroformate under controlled conditions to preclude demethylation of the cladinose dimethylamine group. Preferred protecting groups include acetyl, benzoyl, and trimethylsilyl. A particularly preferred protecting group precursor is trimethylsilyl chloride. Compounds of formula (**2**) can be further elaborated to compounds of formula (**4**) by treatment of the former with R⁷-X¹, wherein X¹ is halide, and base. When derivatizing compounds of formula (**1**), acid can be liberated with the progress of the reaction. For this reason, the reactions described in Scheme 1 are usually run with at least a stoichiometric amount of base present. Examples of such bases include pyridine, diisopropylethylamine, and TEA. Although the solvent used in the reaction is not particularly limited, a solvent which is not reactive with the starting materials and in which the starting materials are both soluble is generally used. Examples of such solvents include acetone, acetonitrile, diethyl ether, DCM, chloroform, ethyl acetate, THF, dioxane, or mixtures thereof. Other factors determine the product disposition, as well. For example, treatment of compounds of formula (**1**) with one equivalent of a hydroxy protecting group precursor and base at room temperature usually results in compounds of formula (**2**). Treatment of compounds of formula (**1**) with two equivalents of a hydroxy protecting group precursor and base with catalytic DMAP at room temperature or elevated temperature usually results in compounds of formula (3). The reaction time is generally 30 minutes to 18 hours and can be selected depending on the desired product disposition.

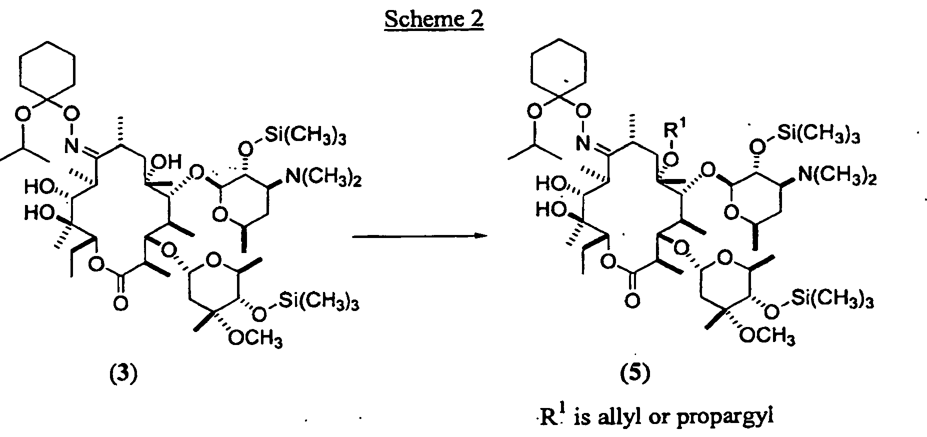

The conversion of compounds of formula (**3**) to compounds of formula (**5**) is shown in Scheme 2. Alkylation of compounds of formula (**3**) can be accomplished by treatment of the former with R¹-X¹ in the presence of base as described in US 5,866,549, and in the experimentals below. An alternative preparation of compounds of formula (I), wherein R¹ is allyl (-C₃-alkenyl), is treatment of compounds of formula (I) with R¹-X², wherein R¹ is allyl (-C₃-alkenyl) or a substituted alkyl and X² is a *tert*-butyl carbonate moiety, in the presence of a catalyst such as Pd₂(dba)₃, as described in Example 1, Step 1(c). Although the solvent used in these reactions is not particularly limited, a solvent which is not reactive with the starting materials and in which the starting materials are both soluble is generally used. Examples of such solvents include diethyl ether, DME, THF, dioxane, or mixtures thereof. When employing R¹-X¹ to derivatize the 6-position, acid is liberated with the progress of the reaction, so it is preferable to run the reaction in the presence of a suitable deacidifying agent. For this reason, the reaction is usually run in with at least a stoichiometric amount of base present. Examples of such bases include pyridine, diisopropylethylamine, TEA, potassium hydroxide, cesium hydroxide, tetraalkylammonium hydroxide, sodium hydride, potassium hydride, and alkali metal alkoxides such as potassium isopropoxide, potassium *tert*-butoxide, and potassium isobutoxide. The reactions generally proceed at room temperature but can be run at lower or elevated temperatures, as needed. The reaction time is generally 30 minutes to 18 hours and can be selected depending on the types of method chosen.

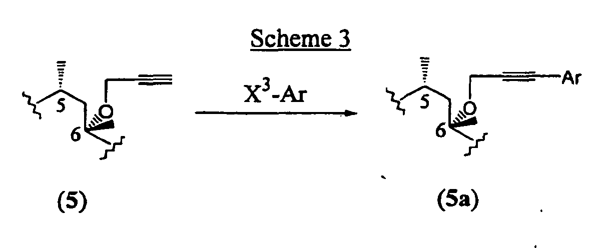

Compounds of formula (**5**), wherein R¹ is propargyl, can be further elaborated to compounds of formula (**5a**) by a number of general routes. A preferred general route is shown in Scheme 3. The 6-O propargyl group can be reacted with groups such as X³-Ar wherein Ar is an unsubstituted or a substituted aryl group or heteroaryl group, respectively, and X³ is one of any number of covalent bond precursors such as halides (preferably bromide and iodide) and sulfonates. The coupling reactions are performed in the presence of Pd(II) or Pd(0) catalysts with promoters such as phosphines (preferably triphenylphosphine), arsines (preferably triphenylarsine), amines (preferably pyridine and triethylamine), and inorganic bases (preferably potassium carbonate or cesium fluoride) in polar, aprotic solvents such as benzene, toluene, DMF, DMSO, DME, acetonitrile THF, or mixtures thereof at temperatures from about room temperature to about 150 °C, depending on the coupling method chosen and the nature of X³.

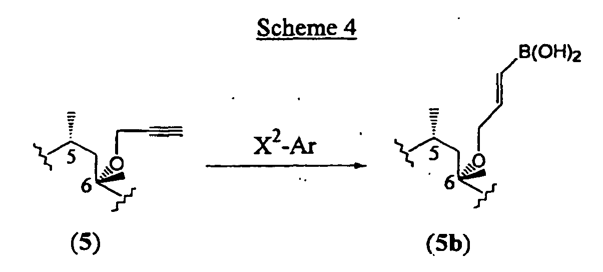

As shown in Scheme 4, compounds of formula (**5**), wherein R¹ is propargyl, can be still further elaborated intermediates of formula (**5b**) with borane-THF in aprotic solvents at temperatures from about -20 °C to about room temperature to provide vinyl boronic acid derivatives. Intermediates (**5b**) then be reacted under Suzuki conditions with X³-Ar reagents, catalysts, and promoters described in Scheme 3 to provide additional compounds of formula (**5**). A thorough discussion of Suzuki conditions is provided in Chemical Reviews, 1995, Vol. 95, No.7, 2457-2483.

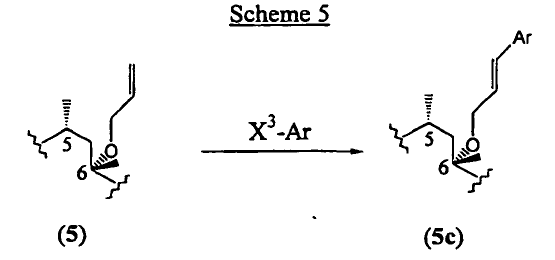

As shown in Scheme 5, compounds of formula (**5**) can be still even further elaborated to compounds of formula (**5c**) by treatment with to X³-Ar reagents under Heck conditions, the conditions of which are described in Larock (op. cit.) and in US 5,866,549 (Example 1 steps 1a-g and Example 102, steps 120a-c).

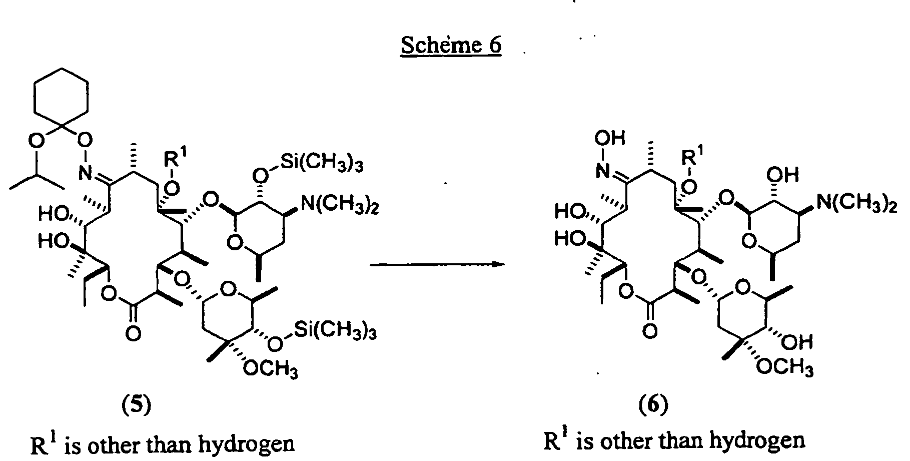

As shown in Scheme 6, the conversion of compounds of formula (**5**) to compounds of formula (**6**) can be accomplished by treatment of the former with an acid such as HCl, HBr, acetic acid, or TFA. Although the solvent used in these reactions is not particularly limited, a solvent which is not reactive with the starting material and in which the starting material and the acid are both soluble is generally used. Examples of such solvents include acetonitrile, DME, THF, dioxane, or mixtures thereof. The preferred conditions for the deprotection of the 2'- and 4"-hydroxy groups of the desosamine and cladinose rings, respectively, (acetic acid in acetonitrile and water) usually result in concomitant removal of the 1-isopropoxycyclohexyl group provide the unalkylated oxime (=N-OH) at C-9.

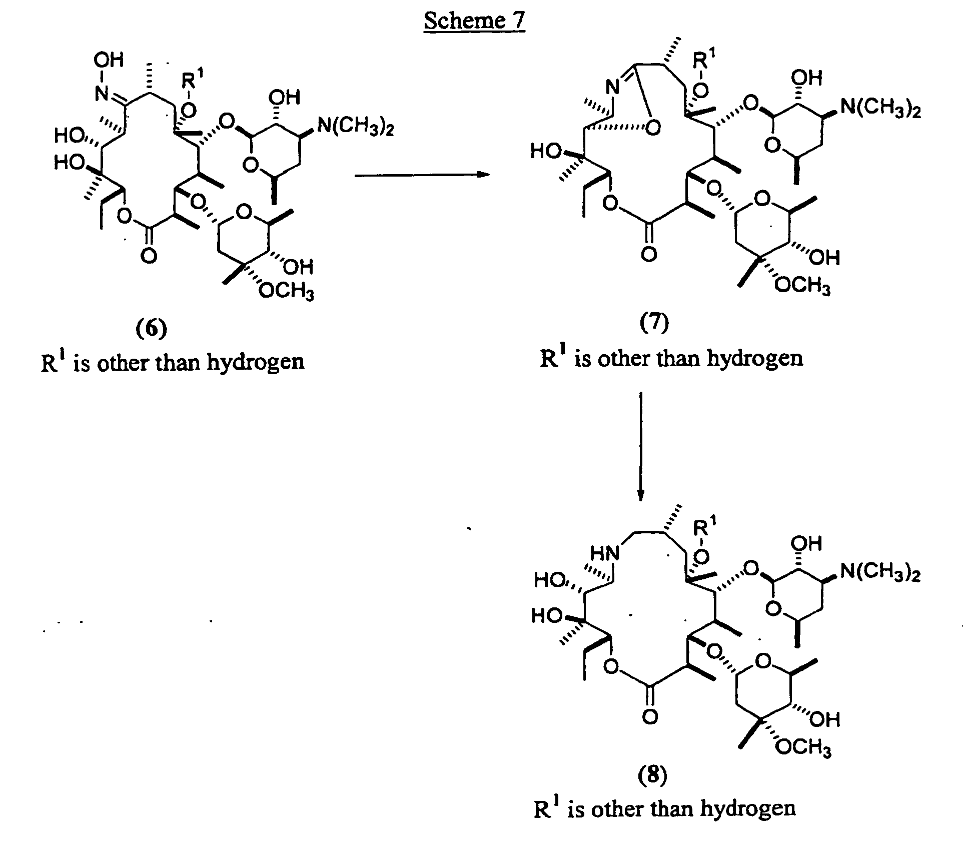

Synthesis of compounds of formula (**7**) and the conversion of compounds of formula (**7**) to compounds of formula (**8**) is shown in Scheme 7. Compounds of formula (**6**) can be treated with oxime activating agents to provide compounds of formula (**7**). Especially preferred oxime activating agents are sulfonyl halides such as para-toluenesulfonyl chloride, methanesulfonyl chloride, para-bromosulfonyl chloride, and para-bromosulfonyl chloride. When using sulfonyl halides to activate oximes, acid is liberated with the progress of the reaction, so it is preferable to run the reaction in the presence of a suitable deacidifying agent. For this reason, the reaction is run with at least a stoichiometric amount of base present. Examples of such bases include pyridine, diisopropylethylamine, TEA, NaHCO₃, Na₂CO₃, KHCO₃, and K₂CO₃. Conversion of compounds of formula (**7**) to compounds of formula (**8**) can be achieved by treatment of the former with reducing agents such as PtO₂, borane in tetrahydrofuran, borane dimethylsulfide, sodium cyanoborohydride, or sodium borohydride optionally in the presence of an acid such as TiCl₄, CoCl₂, AlCl₃, methanesulfonic acid, or acetic acid. In a particularly preferred embodiment, compounds of formula (**6**) are treated with para-toluenesulfonyl chloride and pyridine in THF to provide compounds of formula (**7**) which are treated with NaBH₃CN and acetic acid to provide compounds of compounds of formula (**8**). Although the solvent used in these reactions is not particularly limited, a solvent which is not reactive with the starting materials and in which the starting materials are both soluble is generally used. Examples of such solvents include acetone, water, acetonitrile, diethyl ether, DME, DCM, chloroform, DMF, DMA, ethyl acetate, THF, dioxane, N-methylpyrrolidinone, DMSO, diethylsulfoxide, HMPA, or mixtures thereof The reactions generally proceed at room temperature but can be run at lower temperatures. The reaction time is generally 30 minutes to 18 hours and can be selected depending on the types of starting materials and reaction temperature.

The compounds and processes of the present invention will be better understood in connection with the following examples, which are intended as an illustration of and not a limitation upon the scope of the invention.

Clarithromycin (3-O-cladinosyl-5-O-desosaminyl-6-O-methyl-erythronolide A) was obtained from Abbott Laboratories. All other starting materials not mentioned specifically by example were purchased from Aldrich Chemical Company (Milwaukee, WI).

### Example 1

### Compound of formula (7) from Scheme 7: R¹ is -CH₂CH=CH₂, R⁴ is hydrogen, R⁷ is hydrogen, R⁸ is hydrogen

### Step 1a: Compound (1) from Scheme 1

The desired product was prepared as described in US 4,990,602, Examples (1) and (2), and substituting 1-cyclohexen-1-yl isopropyl ether for 2-methoxypropene.

### Step 1b: allyl tert-butyl carbonate

The desired product was prepared as described in the Canadian Journal of Chemistry, 1985, Vol.63, pp.153-162.

### Step 1c: Compound (5) from Scheme 2: R¹ is -CH₂CH=CH₂, R⁴ is hydrogen, R⁵ is hydrogen, R⁷ is -Si(CH₃)₃, R⁸ is -Si(CH₃)₃, R^{x} is iso-propyl, R^{y} and R^{z} together are cyclohexyl

A mixture of Pd₂(dba)₃ (0.120 g, 0.131mmol) and dppb (111 mg, 0.259 mmol) was treated with the product from Step 1a (azeotropically dried with toluene, 25.0 g, 24.23 mmol) and the product from Step 1b (4.6 g, 29.1 mmol) in THF (100 mL), heated to reflux for 30 minutes, cooled, and concentrated. The concentrate was dissolved in ethyl acetate (250 mL), and the resulting solution was washed with half-saturated Na₂CO₃ and brine, dried (Na₂SO₄), filtered, and concentrated to provide 25.2 g of the desired product of sufficient purity for use without further purification.
MS (DCI/NH₃) *m*/*z* 1073 (M+H)⁺.

### Step 1d: Compound (6) from Scheme 6: R¹ is -CH₂CH=CH₂, R⁴ is hydrogen, R⁵ is hydrogen, R⁷ is hydrogen. R⁸ is hydrogen, R^{x} is hydrogen

A suspension of the product from Step 1c (25.2 g, 23.5 mmol) in acetonitrile (240 mL) and water (50 mL) was treated with glacial acetic acid (70 mL), and the resulting solution was stirred at room temperature for 3 days and concentrated: The concentrate was dissolved in DCM (100 mL), and the resulting solution was washed sequentially with half-saturated Na₂CO₃ (until the pH of the wash was 10), water, and brine, dried (Na₂SO₄), filtered, and concentrated to provide 17.4 g of the desired product of sufficient purity for use without further purification.
MS (DCI/NH₃) *m*/*z* 790 (M+H)⁺.

### Step 1e: Compound of formula (7): R¹ is -CH₂CH=CH₂, R⁴ is hydrogen, R⁷ is hydrogen, R⁸ is hydrogen

A solution of the product from Step 1d (7.0 g, 8.88 mmol) in pyridine (90 mL) at 0 °C was treated over 30 minutes with a solution of para-toluenesulfonyl chloride (3.39 g, 17.77 mmol) in THF (90 mL), stirred for 2.5 hours, heated to 40 °C for 20 hours, cooled, and treated with ethyl acetate (200 mL). The resulting solution was washed sequentially with half-saturated Na₂CO₃, water, and brine, dried (Na₂SO₄), filtered, and concentrated. The concentrate was purified by flash column chromatography on silica gel with 96:4 acetone/TEA to provide 2.86 g of the desired product.
MS (DCI/NH₃) *m*/*z* 771 (M+H)⁺.

### Example 2

### Compound of formula (I): R¹ is -CH₂CH=CH₂, R⁴ is hydrogen. R⁵ is hydrogen, R⁶ is hydrogen, R⁷ is hydrogen, R⁸ is hydrogen

A solution of Example 1 (100 mg, 0.13 mmol) and one crystal of bromocresol green in methanol (1 mL) at room temperature was treated with glacial acetic acid until the color of the solution changed from blue to yellow, treated a first time with NaBH₃CN (16 mg, 0.26 mmol), stirred at room temperature for 18 hours, treated a second time with NaBH₃CN (16 mg, 0.260 mmol), stirred for 4 hours, treated a third time with NaBH₃CN (32 mg, 0.52 mmol), stirred for 3 hours, and treated a fourth time with NaBH₃CN (32 mg, 0.52 mmol), and stirred for 16 hours, treated with 1M NaOH (5 mL), and extracted with MTBE. The extract was washed with brine, dried (Na₂SO₄), filtered, and concentrated. The concentrate was purified by flash column chromatography on silica gel with 9:1:0.5 DCM/methanol/concentrated NH₄OH to provide 15.1 mg of the desired product.
MS (DCI/NH₃) *m*/*z* 775 (M+H)⁺.

## Claims

1. A compound of formula (I) 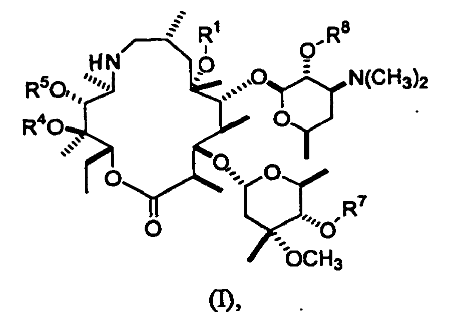
or pharmaceutically acceptable salts or prodrugs thereof, wherein
R¹ is selected from the group consisting of
(1) -C₃-C₁₂-alkenyl,
and
(2) -C₃-C₁₂-alkynyl,
wherein (1)-(2) can be optionally substituted with one, two, or three substituents independently selected from the group consisting of
(a) halogen,
(b) -OR⁹, wherein R⁹ is selected from the group consisting of
(i) hydrogen,
(ii) -C₁-C₁₂-alkyl,
(iii) -C₂-C₁₂-heteroalkyl,
wherein (ii) and (iii) can be optionally substituted with one, two, or three substituents independently selected from the group consisting of
(1') aryl,
(2') substituted aryl,
(3') heteroaryl,
and
(4') substituted heteroaryl,
(iv) aryl,
(v) substituted aryl,
(vi) heteroaryl,
and
(xv) substituted heteroaryl,
(c) -NR¹¹R¹², wherein R¹¹ and R¹² are independently selected from the group consisting of
(i) hydrogen,
(ii) -C₁-C₁₂-alkyl,
(iii) -C₂-C₁₂-heteroalkyl,
wherein (ii) and (iii) can be optionally substituted with one, two, or three substituents independently selected from the group consisting of
(1') aryl,
(2') substituted aryl,
(3') heteroaryl,
and
(4') substituted heteroaryl,
(iv) aryl,
(v) substituted aryl,
(vi) heteroaryl,
and
(xv) substituted heteroaryl,
or
R¹¹ and R¹², together with the atom to which they are attached, form a heterocycloalkyl ring, wherein the heterocycloalkyl ring can be optionally substituted,
(d) =N-O-R⁹, wherein R⁹ is defined above,
(e) aryl,
(f) substituted aryl,
(g) heteroaryl,
(h) substituted heteroaryl,
(i) -C₃-C₈-cycloalkyl,
(j) substituted -C₃-C₈-cycloalkyl,
(k) heterocycloalkyl,
(l) substituted heterocycloalkyl,
(m) NHC(O)R⁹, wherein R⁹ is defined above,
(n) -NEC(O)OR¹⁰, wherein R¹⁰ is selected from the group consisting of
(i) -C₁-C₁₂-alkyl,
(ii) -C₁-C₁₂-heteroalkyl,
wherein (i) and (ii) can be optionally substituted with one, two, or three substituents independently selected from the group consisting of
(1') aryl,
(2') substituted aryl,
(3') heteroaryl,
and
(4') substituted heteroaryl,
(iii) aryl,
(iv) substituted aryl,
(v) heteroaryl,
and
(vi) substituted heteroaryl,
(o) -NHC(O)NR¹¹R¹², wherein R¹¹ and R¹² are defined above,
(p) -OC(O)R¹⁰, wherein R¹⁰ is defined above,
(q) -OC(O)OR¹⁰, wherein R¹⁰ is defined above,
(r) -OC(O)NR¹¹R¹², wherein R¹¹ and R¹² are defined above,
(s) -C(O)R⁹, wherein R⁹ is defined above,
(t) -CO₂R⁹, wherein R⁹ is defined above,
and
(u) -C(O)NR¹¹R¹², wherein R¹¹ and R¹² are defined above;
R⁴ and R⁵ are hydrogen;
or
R⁴ and R⁵ together are -C(O)- or -(CH₂)ₓ-, wherein x is one, two, or three;
R⁷ is selected from the group consisting of
(1) hydrogen,
(2) -C₁-C₁₂-alkyl,
(3) -C₃-C₁₂-alkenyl,
(4) -C₃-C₁₂-alkynyl,
(5) -C₂-C₁₂-heteroalkyl,
(6) -C₄-C₁₂-heteroalkenyl,
(7) -C₄-C₁₂-heteroalkynyl,
wherein (2)-(7) can be optionally substituted with one, two, or three substituents independently selected from the group consisting of
(a) halo,
(b) hydroxy,
(c) -NR¹¹R¹², wherein R¹¹ and R¹² are defined above,
(d) aryl,
(e) substituted aryl,
(f) heteroaryl,
and
(g) substituted heteroaryl,
(8) -C(O)R⁹, wherein R⁹ is defined above,
(9) -CO₂R⁹, wherein R⁹ is defined above,
and
(11) -C(O)NR¹¹R¹², wherein R¹¹ and R¹² are defined above;
and
R⁸ is hydrogen or a hydroxy protecting group.

2. A compound according to Claim 1, wherein R⁴ and R⁵ are hydrogen.

3. A compound according to Claim 1, wherein R⁴ and R⁵ together are -C(O)-.

4. A compound according to Claim 1, wherein R¹ is -CH₂-CH=CH₂ or -CH₂-CH=CH-(3-quinolinyl).

5. A compound according to Claim 4, wherein R¹ is -CH₂-CH=CH₂.

6. A compound according to Claim 4, wherein R¹ is -CH₂-CH=CH-(3-quinolinyl).

7. A method for preparing a compound according to Claim 1, the method comprising
(a) reacting a compound of formula (Ia) 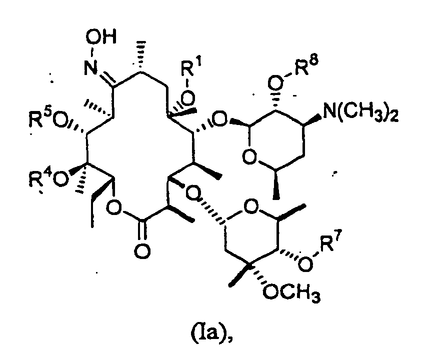
wherein R¹, R⁴, R⁵, R⁷, and R⁸ are as defined in Claim 1,
with an oxime activating agent;
(b) reacting the product from step (a) with a reducing agent;
and
(c) optionally deprotecting the product from step (b).

8. The method according to Claim 7, wherein the oxime activating agent is a sulfonyl halide.

9. The method according to Claim 8, wherein the sulfonyl halide is para-toluenesulfonyl chloride, methanesulfonyl chloride, para-bromosulfonyl chloride, or para-bromosulfonyl chloride.

10. The method according to Claim 7, wherein the reducing agent is borane in tetrahydrofuran, borane dimethyl sulfide, sodium cyanoborohydride, or sodium borohydride, optionally in the presence of an acid.

11. The method according to Claim 10, wherein the reducing agent is sodium cyanoborohydride in the presence of acid.

12. The method according to Claim 11, wherein the acid is acetic acid.

13. A pharmaceutical composition comprising a pharmaceutically effective amount of a compound of Claim 1 in combination with a pharmaceutically acceptable carrier.

14. Use of a compound of Claim 1 for the manufacture of a medicament for treating a bacterial infection in a mammal in need of such treatment.

15. A compound of Claim 1 for use as a therapeutic agent.

16. A compound of Claim 1 wherein R¹ is -CH₂CH=CH₂, R⁴ is hydrogen, R⁵ is hydrogen, R⁷ is hydrogen, and R⁸ is hydrogen.

## Patentansprüche

1. Eine Verbindung von Formel (I) 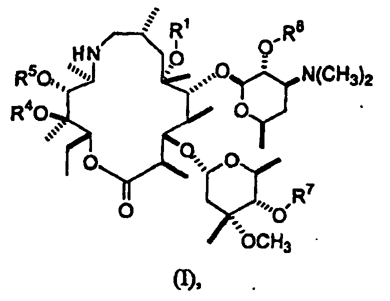
oder pharmazeutisch verträgliche Salze oder Prodrugs davon, worin
R¹ gewählt ist aus der Gruppe bestehend aus
(1) -C₃-C₁₂-Alkenyl,
und
(2) -C₃-C₁₂-Alkinyl,
worin (1)-(2) wahlweise substituiert sein kann mit ein, zwei oder drei Substituenten unabhängig gewählt aus der Gruppe bestehend aus
(a) Halogen,
(b) -OR⁹, worin R⁹ gewählt ist aus der Gruppe bestehend aus
(i) Wasserstoff,
(ii) -C₁-C₁₂-Alkyl,
(iii) -C₂-C₁₂-Heteroalkyl,
worin (ii) und (iii) wahlweise substituiert sein können mit ein, zwei oder drei Substituenten unabhängig gewählt aus der Gruppe bestehend aus
(1') Aryl,
(2') substituiertem Aryl,
(3') Heteroaryl,
und
(4') substituiertem Heteroaryl,
(iv) Aryl,
(v) substituiertem Aryl,
(vi) Heteroaryl,
und
(xv) substituiertem Heteroaryl,
(c) -NR¹¹R¹², worin R¹¹ und R¹² unabhängig gewählt sind aus der Gruppe bestehend aus
(i) Wasserstoff,
(ii) -C₁-C₁₂-Alkyl,
(iii) -C₂-C₁₂-Heteroalkyl,
worin (ii) und (iii) wahlweise substituiert sein können mit ein, zwei oder drei Substituenten unabhängig gewählt aus der Gruppe bestehend aus
(1') Aryl,
(2') substituiertem Aryl,
(3') Heteroaryl,
und
(4') substituiertem Heteroaryl,
(iv) Aryl,
(v) substituiertem Aryl,
(vi) Heteroaryl,
und
(xv) substituiertem Heteroaryl,
oder
R¹¹ und R¹², zusammengenommen mit dem Atom, an welches sie gebunden sind, bilden einen Hetercycloalkylring, worin der
Heterocycloalkylring wahlweise substituiert sein kann,
(d) =N-O-R⁹, worin R⁹ oben definiert ist,
(e) Aryl,
(f) substituiertem Aryl,
(g) Heteroaryl,
(h) substituiertem Heteroaryl,
(i) -C₃-C₈-Cycloalkyl,
(j) substituiertem -C₃-C₈-Cycloalkyl
(k) Heterocycloalkyl,
(1) substituiertem Heterocycloalkyl,
(m) -NHC (O) R⁹, worin R⁹ oben definiert ist,
(n) -NHC (O) OR¹⁰, worin R¹⁰ gewählt ist aus der Gruppe bestehend aus
(i) -C₁-C₁₂-Alkyl,
(ii) -C₁-C₁₂-Heteroalkyl,
worin (i) und (ii) wahlweise substituiert sein können mit ein, zwei oder drei Substituenten unabhängig gewählt aus der Gruppe bestehend aus
(1') Aryl,
(2') substituiertem Aryl,
(3') Heteroaryl,
und
(4') substituiertem Heteroaryl,
(iii) Aryl,
(iv) substituiertem Aryl,
(v) Heteroaryl,
und
(vi) substituiertem Heteroaryl,
(o) -NHC(O)NR¹¹R¹², worin R¹¹ und R¹² oben definiert sind,
(p) -OC(O)R¹⁰, worin R¹⁰ oben definiert ist,
(q) -OC(O)OR¹⁰, worin R¹⁰ oben definiert ist,
(r) -OC(O)NR¹¹R¹², worin R¹¹ und R¹² oben definiert sind,
(s) -C (O) R⁹, worin R⁹ oben definiert ist,
(t) -CO₂R⁹, worin R⁹ oben definiert ist,
und
(u) -C(O)NR¹¹R¹², worin R¹¹ und R¹² oben definiert sind;
R⁴ und R⁵ sind Wasserstoff;
oder
R⁴ und R⁵ zusammen sind -C(O)- oder -(CH₂)ₓ-, worin x eins, zwei oder drei ist;
R⁷ ist gewählt aus der Gruppe bestehend aus
(1) Wasserstoff,
(2) -C₁-C₁₂-Alkyl,
(3) -C₃-C₁₂-Alkenyl,
(4) -C₃-C₁₂-Alkinyl,
(5) -C₂-C₁₂-Heteroalkyl,
(6) -C₄-C₁₂-Heteroalkenyl,
(7) -C₄-C₁₂-Heteroalkinyl, worin (2)-(7) wahlweise substituiert sein können mit ein, zwei oder drei Substituenten unabhängig gewählt aus der Gruppe bestehend aus
(a) Halo,
(b) Hydroxy,
(c) -NR¹¹R¹², worin R¹¹ und R¹² oben definiert sind,
(d) Aryl,
(e) substituiertem Aryl,
(f) Heteroaryl,
und
(g) substituiertem Heteroaryl,
(8) -C (O) R⁹, worin R⁹ oben definiert ist,
(9) -CO₂R⁹, worin R⁹ oben definiert ist,
und
(11) -C(O)NR¹¹R¹², worin R¹¹ und R¹² oben definiert sind;
und
R⁸ ist Wasserstoff oder eine Hydroxyschutzgruppe.

2. Eine Verbindung gemäß Anspruch 1, worin R⁴ und R⁵ Wasserstoff sind.

3. Eine Verbindung gemäß Anspruch 1, worin R⁴ und R⁵ zusammen - C(O) - sind.

4. Eine Verbindung gemäß Anspruch 1, worin R¹ -CH₂-CH=CH₂ oder - CH₂-CH=CH-(3-Chinolinyl) ist.

5. Eine Verbindung gemäß Anspruch 4, worin R¹ -CH₂-CH=CH₂ ist.

6. Eine Verbindung gemäß Anspruch 4, worin R¹ -CH₂-CH=CH-(3-Chinolinyl) ist.

7. Ein Verfahren zur Herstellung einer Verbindung gemäß Anspruch 1, wobei das Verfahren folgendes umfasst:
(a) Reagieren einer Verbindung von Formel (Ia) 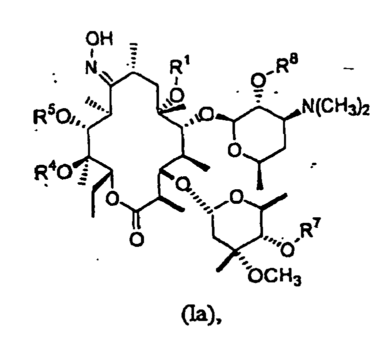
worin R¹, R⁴, R⁵, R⁷ und R⁸ wie in Anspruch 1 definiert sind,
mit einem Oxim aktivierenden Wirkstoff;
(b) Reagieren des Produkts aus Schritt (a) mit einem Reduktionsmittel;
und
(c) wahlweise Entschützen des Produkts aus Schritt (b).

8. Das Verfahren gemäß Anspruch 7, worin der Oxim aktivierende Wirkstoff ein Sulfonylhalogenid ist.

9. Das Verfahren gemäß Anspruch 8, worin das Sulfonylhalogenid para-Toluensulfonylchlorid, Methansulfonylchlorid, para-Bromsulfonylchlorid oder para-Bromsulfonylchlorid ist.

10. Das Verfahren gemäß Anspruch 7, worin das Reduktionsmittel Boran in Tetrahydrofuran, Boran-Dimethylsulfid, Natriumcyanoborhydrid oder Natriumborhydrid ist, wahlweise in der Anwesenheit von Säure.

11. Das Verfahren gemäß Anspruch 10, worin das Reduktionsmittel Natriumcyanoborhydrid in der Anwesenheit von Säure ist.

12. Das Verfahren gemäß Anspruch 11, worin die Säure Essigsäure ist.

13. Eine pharmazeutische Zusammensetzung, die eine pharmazeutisch wirksame Menge einer Verbindung von Anspruch 1, in Kombination mit einem pharmazeutisch verträglichen Träger umfaßt.

14. Verwendung einer Verbindung von Anspruch 1 für die Herstellung eines Medikaments zur Behandlung einer bakteriellen Infektion in einem Säugetier, das eine solche Behandlung benötigt.

15. Eine Verbindung von Anspruch 1 für die Verwendung als ein therapeutischer Wirkstoff.

16. Eine Verbindung von Anspruch 1, worin R¹ -CH₂CH=CH₂ ist, R⁴ Wasserstoff ist, R⁵ Wasserstoff ist, R⁷ Wasserstoff ist und R⁸ Wasserstoff ist.

## Revendications

1. Composé de formule (I) 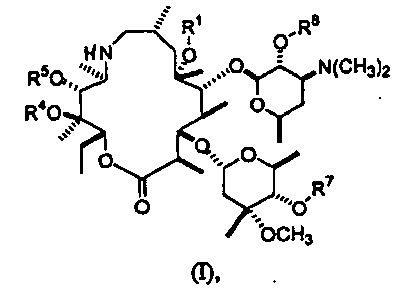
ou sels ou précurseurs de médicament pharmaceutiquement acceptables de celui-ci, où
R¹ est choisi dans le groupe constitué par
(1) un alcényle en C₃-C₁₂,
et
(2) un alcynyle en C₃-C₁₂,
où (1) et (2) peuvent être substitués, de façon optionnelle, par un, deux ou trois
substituants choisis de façon indépendante dans le groupe constitué par
(a) un atome d'halogène ;
(b) -OR⁹, où R⁹ est choisi dans le groupe constitué par
(i) l'atome d'hydrogène,
(ii) un alkyle en C₁-C₁₂,
(iii) un hétéroalkyle en C₂-C₁₂,
où (ii) et (iii) peuvent être substitués, de façon optionnelle, par un, deux ou trois substituants choisis de façon indépendante dans le groupe constitué par
(1') un aryle,
(2') un aryle substitué,
(3') un hétéroaryle,
et
(4') un hétéroaryle substitué,
(iv) un aryle,
(v) un aryle substitué,
(vi) un hétéroaryle,
et
(vii) un hétéroaryle substitué,
(c) -NR¹¹R¹², où R¹¹ et R¹² sont choisis de façon indépendante dans le groupe constitué par
(i) l'atome d'hydrogène,
(ii) un alkyle en C₁-C₁₂,
(iii) un hétéroalkyle en C₂-C₁₂,
où (ii) et (iii) peuvent être substitués, de façon optionnelle, par un, deux ou trois substituants choisis de façon indépendante dans le groupe constitué par
(1') un aryle,
(2') un aryle substitué,
(3') un hétéroaryle,
et
(4') un hétéroaryle substitué,
(iv) un aryle,
(v) un aryle substitué,
(vi) un hétéroaryle,
et
(vii) un hétéroaryle substitué,
ou
R¹¹ et R¹², pris avec l'atome auquel ils sont attachés, forment un cycle hétérocycloalkyle, où le cycle hétérocycloalkyle peut être substitué de façon optionnelle,
(d) =N-O-R⁹, où R⁹ est tel que défini ci-dessus,
(e) un aryle,
(f) un aryle substitué,
(g) un hétéroaryle,
(h) un hétéroaryle substitué,
(i) un cycloalkyle en C₃-C₈,
(j) un cycloalkyle en C₃-C₈ substitué,
(k) un hétérocycloalkyle,
(1) un hétérocycloalkyle substitué,
(m) -NHC(O)R⁹, où R⁹ est tel que défini ci-dessus,
(n) -NHC(O)OR¹⁰, où R¹⁰ est choisi dans le groupe constitué par,
(i) un alkyle en C₁-C₁₂,
(ii) un hétéroalkyle en C₁-C₁₂,
où (i) et (ii) peuvent être substitués, de façon optionnelle, par un, deux ou trois substituants choisis de façon indépendante dans le groupe constitué par
(1') un aryle,
(2') un aryle substitué,
(3') un hétéroaryle,
et
(4') un hétéroaryle substitué,
(iii) un aryle,
(iv) un aryle substitué,
(v) un hétéroaryle,
et
(vi) un hétéroaryle substitué,
(o) -NHC(O)NR¹¹R¹², où R¹¹ et R¹² sont tels que définis ci-dessus,
(p) -OC(O)R¹⁰, où R¹⁰ est tel que défini ci-dessus,
(q) -OC(O)OR¹⁰, où R¹⁰ est tel que défini ci-dessus,
(r) -OC(O)NR¹¹R¹², où R¹¹ et R¹² sont tels que définis ci-dessus,
(s) -C(O)R⁹, où R⁹ est tel que défini ci-dessus,
(t) -CO₂R⁹, où R⁹ est tel que défini ci-dessus,
et
(u) -C(O)NR¹¹R¹², où R¹¹ et R¹² sont tels que définis ci-dessus ;
R⁴ et R⁵ sont des atomes d'hydrogène ;
ou
R⁴ et R⁵ pris ensemble sont -C(O)- ou -(CH₂)ₓ-, où x est égal à un, deux ou trois ;
R⁷ est choisi dans le groupe constitué par
(1) un atome d'hydrogène,
(2) un alkyle en C₁-C₁₂,
(3) un alcényle en C₃-C₁₂,
(4) un alcynyle en C₃-C₁₂,
(5) un hétéroalkyle en C₂-C₁₂,
(6) un hétéroalcényle en C₄-C₁₂,
(7) un hétéroalcynyle en C₄-C₁₂,
où (2) à (7) peuvent être substitués, de façon optionnelle, par un, deux ou trois substituants choisis de façon indépendante dans le groupe constitué par
(a) un halogéno,
(b) un hydroxyle,
(c) -NR¹¹R¹², où R¹¹ et R¹² sont tels que définis ci-dessus,
(d) un aryle,
(e) un aryle substitué,
(f) un hétéroaryle,
et
(g) un hétéroaryle substitué,
(8) -C(O)R⁹, où R⁹ est tel que défini ci-dessus,
(9) -CO₂R⁹, où R⁹ est tel que défini ci-dessus,
et
(10) -C(O)NR¹¹R¹², où R¹¹ et R¹² sont tels que définis ci-dessus ;
et
R⁸ est un hydrogène ou un groupe protecteur d'hydroxyle.

2. Composé selon la revendication 1, où R⁴ et R⁵ sont des atomes d'hydrogène.

3. Composé selon la revendication 1, où R⁴ et R⁵ constituent, ensemble, -C(O)-.

4. Composé selon la revendication 1, où R¹ est -CH₂-CH=CH₂ ou -CH₂-CH=CH-(3-quinoléinyle).

5. Composé selon la revendication 4, où R¹ est -CH₂-CH=CH₂.

6. Composé selon la revendication 4, où R¹ est -CH₂-CH=CH-(3-quinoléinyle).

7. Méthode pour la préparation d'un composé selon la revendication 1, la méthode comprenant
(a) la réaction d'un composé de formule (Ia) 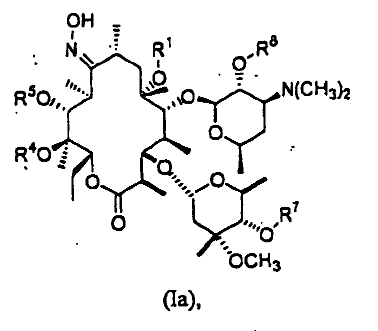
où R¹, R⁴, R⁵, R⁷ et R⁸ sont tels que définis dans la revendication 1,
avec un agent activant les oximes ;
(b) la réaction du produit de l'étape (a) avec un agent réducteur; et
(c) de façon optionnelle la déprotection du produit de l'étape (b).

8. Méthode selon la revendication 7, dans laquelle l'agent activant les oximes est un halogénure de sulfonyle.

9. Méthode selon la revendication 8, dans laquelle l'halogénure de sulfonyle est le chlorure de para-toluènesulfonyle, le chlorure de méthanesulfonyle, le chlorure de para-bromosulfonyle ou le chlorure de para-bromosulfonyle.

10. Méthode selon la revendication 7, dans laquelle l'agent réducteur est le borane dans le tétrahydrofurane, le borane-sulfure de diméthyle, le cyanoborohydrure de sodium, ou le borohydrure de sodium, de façon optionnelle en présence d'un acide.

11. Méthode selon la revendication 10, dans laquelle l'agent réducteur est le cyanoborohydrure de sodium en présence d'un acide.

12. Méthode selon la revendication 11, dans laquelle l'acide est l'acide acétique.

13. Composition pharmaceutique comprenant une quantité pharmaceutiquement acceptable d'un composé selon la revendication 1, en combinaison avec un support pharmaceutiquement acceptable.

14. Utilisation d'un composé selon la revendication 1, pour la fabrication d'un médicament pour le traitement d'une infection bactérienne chez un mammifère ayant besoin d'un tel traitement.

15. Composé selon la revendication 1 pour son utilisation en tant qu'agent thérapeutique.

16. Composé selon la revendication 1, où R¹ est -CH₂-CH=CH₂, R⁴ est un hydrogène, R⁵ est un hydrogène, R⁷ est un hydrogène et R⁸ est un hydrogène.
